**Europäisches Patentamt**

**European Patent Office**

**Office européen des brevets**

(19)

(11) Veröffentlichungsnummer : **0 423 234 B1**

(12) # EUROPÄISCHE PATENTSCHRIFT

(45) Veröffentlichungstag der Patentschrift :
**11.11.92 Patentblatt 92/46**

(51) Int. Cl.$^5$ : **A61J 15/00, A61M 25/10**

(21) Anmeldenummer : **89908701.9**

(22) Anmeldetag : **03.07.89**

(86) Internationale Anmeldenummer :
**PCT/EP89/00759**

(87) Internationale Veröffentlichungsnummer :
**WO 90/00044 11.01.90 Gazette 90/02**

(54) **MAGENSONDE.**

(30) Priorität : **01.07.88 DE 8808484 U**

(43) Veröffentlichungstag der Anmeldung :
**24.04.91 Patentblatt 91/17**

(45) Bekanntmachung des Hinweises auf die
Patenterteilung :
**11.11.92 Patentblatt 92/46**

(84) Benannte Vertragsstaaten :
**AT BE CH DE FR GB IT LI LU NL SE**

(56) Entgegenhaltungen :
**DE-A- 3 610 091**
**US-A- 3 046 988**
**US-A- 4 185 638**

(73) Patentinhaber : **Roewer, Norbert, Dr.**
**Bei der Lutherbuche 37**
**W-2000 Hamburg 54 (DE)**

(72) Erfinder : **Roewer, Norbert, Dr.**
**Bei der Lutherbuche 37**
**W-2000 Hamburg 54 (DE)**

(74) Vertreter : **Glawe, Delfs, Moll & Partner**
**Patentanwälte**
**Postfach 26 01 62 Liebherrstrasse 20**
**W-8000 München 26 (DE)**

## Beschreibung

Die Erfindung betrifft eine Magensonde mit einem zur Ausleitung von Mageninhalt hinreichend bemessenen Saugschlauch, einem Magenballon der durch Spannung des Sangschlauchs in Anlage am Mageneingang zuhalten ist und einem Nasenstopper zur Fixierung des Spannungszustands des Sangschlauchs in der vorgesehenen Lage. Eine solche Mangensonde ist z.B. aus der US-A-3 046 988 bekannt.

Es ist bis heute nicht gelungen, die Aspiration von saurem Mageninhalt in die Bronchien als Ursache anaesthesiologischer Todesfälle zu eliminieren (Anaesthesist, 1987, 36:599-607). Besonders kritisch sind die Ein- und Ausleitungsphasen der Narkose während des Ein- bzw. Ausführen des Narkosetubus, weil in diesen Phasen die Reizung des Rachenraumes besonders stark ist und der Tubus in der Luftröhre noch nicht bzw. nicht mehr durch eine Manschette verblockt ist, die während des normalen Liegens des Tubus das Eindringen von Mageninhalt in die Bronchien im allgemeinen verhindert (mit Ausnahme von pädiatrischen Tuben, die keine Manschette aufweisen). Erhöhtes Risiko besteht bei nicht nüchternen Patienten (Notfallpatienten) und erhöhtem Druck auf Magen bzw. Darm (Schwangerschalt, Ileus). In diesen Fällen saugt man vor dem Legen und dem Ziehen des Narkosetubus mittels einer normalen Magensonde den Mageninhalt ab. Sicherheit wird dadurch aber nicht erreicht, weil die Entleerung im allgemeinen unvollständig ist bzw. Darminhalt in den Magen zurückgelangen kann. - Die schleichende Aspiration von Mageninhalt kann bei langzeitbeatmeten Intensivpatienten für Lungenentzündung verantwortlich sein.

Diesem Nachteil will eine bekannte Sonde des eingangs genannten Typs (DE-A 24 12 553) dadurch begegnen, daß sie einen Magenballon aufweist, der nach dem Legen der Sonde im Magen gebläht wird und derart am Mageneingang anliegen soll, daß einen Reflux von Mageninhalt verhindert wird. In dieser Lage soll der Ballon dadurch gesichert werden, daß nach dem Blähen des im Magen freiliegenden Magenballons das Gerät zurückgezogen wird, bis ein Widerstand die Anlage des Magenballons am Mageneingang anzeigt. Danach wird eine im Speiseröhrenbereich liegende Manschette gebläht, die die Sonde durch ihre Reibung an der Speiseröhrenwandung in der vorgesehenen Lage sichern und einen zusätzlichen Widerstand gegen Reflux von Mageninhalt bilden soll. Diese Sonde hat sich jedoch nicht durchsetzen können. Wegen der Gefahr einer Perforation darf die Speiseröhrenmanschette nur verhältnismäßig geringen Druck enthalten. Da die Speiseröhre - auch aufgrund von Anomalien - sehr unterschiedlichen Durchmesser aufweisen kann, muß die Speiseröhrenmanschette verhältnismäßig weit ausgeführt sein. Das bedeutet, daß sie bei geringem Blähdruck durch

die Möglichkeit gegensinniger, gleichsam rollender Verformung ihrer Enden zu einer axialen Lagesicherung nicht fähig ist. Durch das Vorhandensein eines äußeren Kontrollballons, der lediglich den Blähzustand der Manschette anzeigt, läßt sich dieser Nachteil nicht vermeiden.

Bekannt ist wie bereits oben erwähnt (US-A 3 046 988) eine Sonde, die einen Magenballon und einen Speiseröhrenballon aufweist, die zur Kompression der oberflächlichen Gefäße in der Speiseröhre und am Mageneingang dienen, um Blutungen zu stillen. Der Zug im Saugschlauch, der die korrekte Lage des Magenballons sichern soll, soll dabei durch einen Nasenstopper aufrechterhalten werden. Jedoch bevorzugt die Praxis für diese Fälle trotz der damit verbundenen Umständlichkeit einen mit dem Saugschlauch verbundenen, gewichtsbelasteten Seilzug (Senkstakensonde, offenkundig vorbenutzt), weil sich damit die Größe der Spannung leicht und zuverlässig bestimmen läßt. Überhaupt sind Nasenstopper nur in solchen Fällen (vornehmlich bei Endotrachealtuben) gebräuchlich, bei denen nur geringe Fixierspannungen auftreten und diese nicht genau eingehalten zu werden brauchen. Das liegt daran, daß der Reibungszustand eines Nasenstoppers an dem zugehörigen Sondenschlauch äußerst unterschiedlich sein kann, je nach dem wie stark mit Sekret die Schlauchoberfläche behaftet ist. Ein Nasenstopper eignet sich daher nach bisheriger Kenntnis nicht für Anwendungsfälle, in denen - wie bei den von der Erfindung als Gattung vorausgesetzten Magensonden - eine vorbestimmte und hohe Spannung aufzubringen ist, zumal die Einhaltung der vorbestimmten Spannung umso wichtiger ist, je höher die Spannung ist.

Jedoch hat die Erfindung erkannt, daß ein Nasenstopper dann sehr wohl zur Aufrechterhaltung einer in engen Grenzen vorbestimmten, hohen Saugschlauchspannung geeignet ist, wenn das erfindungsgemäße Verfahren zum Legen der Sonde angewendet wird. Diese zeichnet sich dadurch aus, daß der Magenballon im Magen bei spannungsfreim Schlauch bis zum Erreichen einer unteren Druckstufe gebläht und danach durch Spannung des Saugschlauchs und die dadurch bewirkte Anlage am Mageneingang auf eine höhere Druckstufe gebracht wird und die Sonde in diesem Zustand mittels des Nasenstoppers fixiert wird. Bei einer erfindungsgemäßen Magensonde wird die Durchführung dieses Verfahrens dadurch möglich, daß das mit dem Magenballon verbundene, separate Lumen des Schlauchs im Außenbereich der Sonde mit einem Druckkontrollgerät verbunden ist, das zur Anzeige von mindestens zwei Druckstufen eingerichtet ist, von denen die niedrigere dem bei ungespanntem Schlauch frei geblähten Zustand des Magenballons und die höhere dem Spannungszustand des Schlauchs zugeordnet ist.

Es ist bekannt, Sondenballons mit äußeren Prüfballons zu verbinden (DE-A 35 09 797, DE-C 33 03 582, DE-A 34 12 553), die aber nur den Blähzustand als solchen, nicht aber den Blähdruck anzeigen. Ferner ist es für besondere Anwendungsfälle bekannt, einen Kontrollballon mit Druckanzeige zu verwenden (DE-A 36 10 091, US-A 4 185 638). Bei Magenballons für gattungsgemäße Geräte war eine solche Druckanzeige bislang nicht sinnvoll, weil sie nichts aussagte über den Anlagedruck, der von der Saugschlauchspannung abhängig ist, und war daher nicht naheliegend. Außerdem besteht folgender grundsätzlicher Unterschied zwischen diesen und der Erfindung. In den bekannten Fällen wird mittels des äußeren Druckkontrollgeräts unmittelbar derjenige Druck kontrolliert, der durch Druckluftzufuhr erzielt werden soll, und diese wird dann abgestellt, wenn der gewünschte Druck gemäß Anzeige des Geräts erreicht ist. Diesem bekannten Gebrauch entspricht im Falle der Erfindung lediglich die untere Druckstufe. Die obere Druckstufe dient demgegenüber nicht der Kontrolle der Druckzufuhr und auch nicht eigentlich zur Kontrolle eines Drucks, sondern zur Kontrolle der Saugschlauchspannung. Es wird damit ein sehr einfaches und genaues Mittel zur Feststellung der Saugschlauchspannung zur Verfügung gestellt, die dann die Verwendung eines einfachen Fixiermittels, nämlich des Nasenstoppers, möglich macht.

Während die Saugschlauchspannung bei Verwendung einer Zugseilanordnung unabhängig von etwaigen Bewegungen des Patienten konstant bleibt, können sich bei Verwendung eine: Nasenstoppers solche Bewegungen auf die Spannung des Saugschlauchs auswirken. Damit die dadurch hervorgerufenen Änderungen gering bleiben, ist der Saugschlauch zweckmäßigerweise zugelastisch.

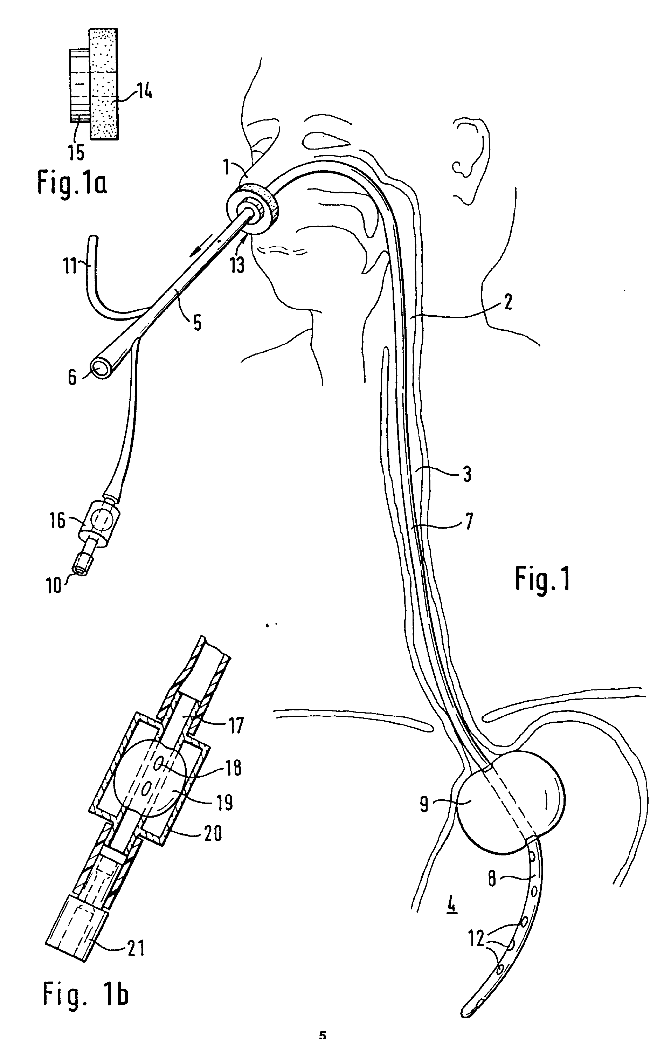

Eine bevorzugte Ausführungsform der Erfindung wird im folgenden näher unter Bezugnahme auf die Zeichnung erläutert, deren einzige Figur die Sonde in der Anwendung veranschaulicht.

In gewohnter Weise ist die Sonde durch Nase 1, Rachen 2, Speiseröhre 3 in den Magen 4 geführt. Sie besteht aus dem Saugschlauch 5 mit einem Sauganschluß 6, einem in der Speiseröhre 3 zu liegen bestimmten Bereich 7 (Speiseröhrenbereich) und einem im Magen 4 zu liegen bestimmten Bereich 8 (Magenbereich). Am oberen Ende des Magenbereichs 8 ist der Ballon 9 in der Art bekannter Manschetten angeordnet, dessen Größenordnung für einen Erwachsenen beispielsweise bei mindestens ca. 20 ml liegt. Er kann durch ein zusätzliches Lumen mit Anschluß 10 und Rückschlagventil 21 aufgeblasen werden, nachdem die Sonde eingeführt worden ist. Ein weiteres Lumen mit Anschlußstück 11 kann für weitere Zwecke, beispielsweise zur Vermeidung eines Unterdrucks im Magen, vorgesehen sein. Der Saugschlauch 5 besitzt im Magenbereich 8 Öffnungen 12 zum Absaugen des Mageninhalts. Ferner trägt der Schlauch einen Nasenstopper 13, der gemäß der vergrößerten Teildarstellung aus einem weichen Schaumgummiring 14 und einem davor angeordneten Fixierung 15 besteht, der eine festere Struktur hat, um den festen Sitz des Stoppers in der eingestellten Position sicherzustellen.

Zumindest während der kritischen Phasen wird die Spannung des Schlauchs durch entsprechende Einstellung des Nasenstoppers so gewählt, daß der Ballon 9 hinreichend dicht am Mageneingang anliegt. Dadurch ist ein Übergang des Mageninhalts in die Speiseröhre weitgehend ausgeschlossen. Da der Saugschlauch im Speiseröhrenbereich 7 keine Öffnungen aufweist, kann der Mageninhalt auch nicht auf dem Wege über den Schlauch in die Speiseröhre gelangen. Im Hinblick auf die Abschlußwirkung des Ballons 9 ist eine ständige Absaugung über die Magensonde nicht notwendig.

Anders als bekannte Magensonden, die nach dem Absaugen des Mageninhalts und vor dem Legen bzw. Ziehen des Narkosetubus im allgemeinen entfernt werden, kann es zweckmäßig sein, die erfindungsgemäße Sonde gerade während dieser kritischen Phasen und ggf. auch intraoperativ an Ort und Stelle zu belassen.

Ferner kann die erfindungsgemäße Sonde bei Langzeitbeatmeten Intensivpatienten zur Verhinderung einer schleichenden Aspiration verwendet werden.

Der Anschluß 10 des zum Magenballon führenden Lumens ist mit einem Druckkontrollgerät 16 versehen. Um ein Rohrstück 17 mit Wandöffnungen 18 ist eine Manschette 19 angeordnet, die zweckmäßigerweise durch ein durchsichtiges Gehäuse 20 geschützt ist, deren Innenraum durch eine nicht dargestellte Öffnung mit dem Atmosphärendruck in Verbindung steht. Wenn kein Differenzdruck an der Manschette 19 herrscht, liegt diese im wesentlichen an dem Rohr 17 an. Der Vergleich des Manschettendurchmessers mit dem des Gehäuses 20 erlaubt eine hinreichend genaue Abschälzung der durch den Aufblaszustand der Manschette 19 angezeigten Druckstufe. Beispielsweise kann die Manschette 19 so dimensioniert sein, daß dann, wenn sie sich eben zu blähen beginnt oder wenn sie den halben Gehäusedurchmesser erreicht hat, der Magenballon denjenigen Druck aufweist, den man bei seiner anfänglichen, freien Blähung im Magen voraussetzen will, während die in der Darstellung angedeutete Anlage der Manschette 19 an dem Gehäuse 20 die Druckstufe anzeigt, die man bei ordnungsgemäßer Anlage des Ballons am Mageneingang wünscht.

Die Sonde wird in der Weise verwendet, daß sie zunächst bei am Saugschlauch anliegender Manschette des Magenballons gelegt wird, wonach durch den Anschluß 10 ein Luftvolumen eingeführt wird, das zur freien Blähung des Ballons im Magen bis zu der unteren von dem Kontrollballon 19 angezeigten Druck führt. Sodann wird durch Zug am Saugschlauch die dargestellte Anlage des Magenballons

am Mageneingang herbeigeführt. Der Anlagedruck erhöht den Innendruck des Magenballons, der von dem Kontrollballon 19 angezeigt wird. Bei einer bestimmten, von dem Kontrollballon angezeigten Druckstufe ist der gewünschte Anlagedruck des Magenballons erreicht und wird der Nasenstopper 13 an die Nase angeschoben, um diesen Zustand zu fixieren. Eine dauernde Kontrolle des gewünschten Zustands ist duch den Kontrollballon gewährleistet. Relativbewegungen zwischen Magen und Nase wirken sich auf die dichte Anlage des Magenballons am Mageneingang nicht wesentlich aus, weil sie durch die Elastizität des Saugschlauchs über dessen große Länge zwischen Magen und Nase ausgeglichen werden.

## Patentansprüche

1. Magensonde mit einem zur Ausleitung von Mageninhalt hinreichend bemessenen Saugschlauch (5), einem Magenballon (9) der durch Spannung des Saugschlauchs in Anlage am Mageneingang zu halten ist, einem Nasenstopper (13) zur Fixierung des Spannungszustands des Saugschlauchs, dadurch gekennzeichnet, daß ein mit dem Magenballon (9) verbundene separate Lumen des Schlauchs im Außenbereich der Sonde mit einem Druckkontrollgerät (16) verbunden ist, das zur Anzeige von mindestens zwei Druckstufen eingerichtet ist, von denen die niedrigere dem bei ungespanntem Schlauch frei geblähten Zustand des Magenballons und die höhere dem Spannungszustand des Schlauchs zugeordnet ist.

2. Magensonde nach Anspruch 1, dadurch gekennzeichnet, daß der Saugschlauch (5) zugelastisch ist.

## Claims

1. Gastric probe comprising a suction tube (5) of adequate size to pass out stomach contents, a stomach balloon (9) held in position against the entrance to the stomach by tensioning the suction tube, and a nasal stopper (13) for fixing the tension state of the suction tube, characterised in that a separate lumen of the suction tube, connected to the stomach balloon (9) is connected to a pressure monitoring device (16) in the external part of the stomach tube, this device being set up to display at least two pressure steps, the free, inflated state of the stomach balloon with the suction tube not under tension being assigned to the lower of these steps, and the state of tension of the suction tube being assigned to the higher step.

2. Gastric probe in accordance with Claim 1, characterised in that the suction tube (5) possesses elasticity of elongation.

## Revendications

1. Sonde gastrique comprenant un tuyau flexible d'aspiration (5) de dimensions suffisante pour évacuer le contenu de l'estomac, un ballon stomacal (9) qui est destiné à être maintenu appliqué contre l'entrée de l'estomac par tension du tuyau flexible d'aspiration, et un arrêt nasal (13) pour fixer l'état de tension du tuyau souple d'aspiration, caractérisée en ce qu'une lumière séparée du tuyau flexible, en communication avec le ballon stomacal (9), est raccordée dans la partie externe de la sonde à un appareil de contrôle de pression (16) qui est réglé de façon à indiquer au moins deux niveaux de pression, parmi lesquels le plus bas correspond à l'état de gonflement libre du ballon stomacal lorsque le tuyau flexible est détendu, et le plus élevé correspond à l'état tendu du tuyau flexible.

2. Sonde gastrique selon la revendication 1, caractérisée en ce que le tuyau souple d'aspiration (5) est élastique à la traction.

Fig. 1a

Fig. 1

Fig. 1b